Europäisches Patentamt

European Patent Office

Office européen des brevets

(19)

(11) Numéro de publication: **0 200 653**
**B1**

(12) **FASCICULE DE BREVET EUROPEEN**

(45) Date de publication du fascicule du brevet: **14.03.90**

(51) Int. Cl.⁵: **C 12 N 15/00, C 12 N 5/00**

(21) Numéro de dépôt: **86400945.1**

(22) Date de dépôt: **29.04.86**

(54) **Cellules hybrides productrices d'un antigène caractéristique du virus de l'hépatite B obtenu à partir d'hépatocytes et de cellules établies de singe procédé pour l'obtention de ces cellules hybrides et application de celles-ci à la production de l'antigène susdit.**

(30) Priorité: **02.05.85 FR 8506706**

(43) Date de publication de la demande:
**05.11.86 Bulletin 86/45**

(45) Mention de la délivrance du brevet:
**14.03.90 Bulletin 90/11**

(84) Etats contractants désignés:
**AT BE CH DE GB IT LI LU NL SE**

(56) Documents cités:
**EP-A-0 062 574**
**EP-A-0 145 589**
**WO-A-82/03087**
**WO-A-85/03946**

(73) Titulaire: **INSTITUT PASTEUR**
**25/28, rue du Docteur Roux**
**F-75015 Paris (FR)**
(73) Titulaire: **CENTRE NATIONAL DE LA RECHERCHE SCIENTIFIQUE (CNRS)**
**15, Quai Anatole France**
**F-75007 Paris (FR)**

(72) Inventeur: **Chenciner Nicole**
**11, quai Bourbon**
**F-75004 Paris (FR)**
Inventeur: **Houssais, Jean-François**
**35, Allée des Comtes de Montford-Aufargis**
**F-78610 Le Perray en Yvelines (FR)**

(74) Mandataire: **Gutmann, Ernest et al**
**S.C. Ernest Gutmann - Yves Plasseraud 67, boulevard Haussmann**
**F-75008 Paris (FR)**

Courier Press, Leamington Spa, England.

**Description**

L'invention est relative à des hybrides cellulaires efficaces pour la production d'un polypeptide ayant les propriétés immunologiques et, de préférence aussi, immunogènes, d'un antigène du virus de l'hépatite B, de préférence, d'un antigène de l'enveloppe de ce virus, plus particulièrement de l'antigène HBS Ag ou d'un fragment de celui-ci, ce polypeptide étant codé par une séquence d'ADN cloné ou clonable dans cet hybride cellulaire. L'invention est également relative à un procédé de production de ces hybrides cellulaires et à l'application de ces derniers à la production dudit polypeptide.

Les hybrides cellulaires de la présente invention entrent dans le cadre de ceux qui ont déjà été décrits dans la demande de brevet européen no. 85400446.2/158546. On rappelle que les hybrides cellulaires du brevet européen, qui sont transformés ou transformables par une séquence d'ADN clonée déterminée, sont caractérisés en ce qu'ils contiennent, d'une part, une partie au moins du patrimoine génétique d'une lignée de cellules primaires naturellement favorables à l'expression de gènes naturels codant pour une protéine constituée par le polypeptide codé par la susdite séquence d'ADN clonée ou contenant dans sa structure une séquence polypeptidique identique ou analogue à celle de ce polypeptide et, d'autre part, un marqueur génétique leur permettant de pousser dans un milieu sélectif ou contenant un principe actif normalement léthal pour les cellules dont l'hybride est issu, mais inactivable par le polypeptide exprimé par ledit marqueur génétique. L'expression "cellules primaires" telle qu'utilisée dans ce qui précède désigne notamment toute cellule prélevée sur un mammifère et connue par sa capacité à être le siège de la production de la protéine ou du polypeptide correspondant à cette protéine dont la production est recherchée. Ces cellules primaires sont caractérisées par le fait qu'elles ne se multiplient guère *in vitro*, que leur prolifération est tout au plus limitée à la production de monocouches, le développement étant ensuite interrompu par "inhibition de contact" ou analogue. Ceci étant, ces cellules peuvent tout de même, en tout état de cause, être maintenues un certain temps en survie dans des milieux appropriés.

L'expression "cellules eucaryotes établies" désigne des cellules eucaryotes, plus particulièrement de mammifères qui peuvent être cultivées *in vitro* et qui sont capables de se multiplier à travers plusieurs générations.

Le procédé selon l'invention de la précédente demande de brevet européen pour former un hybride cellulaire exprimant ou pouvant être rendu capable d'exprimer une séquence d'ADN cloné déterminée était caractérisé:

— en ce que l'on réalise une coculture, dans des conditions propres à permettre leur hybridation, d'une part, de cellules eucaryotes établies, dans lesquelles la susdite séquence d'ADN clonée est exprimable ou exprimée et, d'autre part, de cellules primaires naturellement favorables à l'expression de gènes naturels codant pour une protéine constituée par le polypeptide codé par la susdite séquence d'ADN clonée ou contenant dans sa propre structure une séquence polypeptidique identique ou analogue à celle de ce polypeptide, cette coculture étant réalisée dans un milieu qui n'autorise pas le développement desdites cellules établies, lorsqu'elles ne sont pas complémentées par une séquence génétique appropriée,

— en ce que les cellules primaires mises en jeu dans l'hybridation cellulaire sont aptes ou ont été rendues aptes au préalable à fournir ladite séquence génétique appropriée à au moins une partie des hybrides cellulaires formés dans ledit milieu de culture et en ce que l'on recueille les hybrides cellulaires formés.

L'invention de la susdite demande de brevet européen fournit donc des micro-organismes modifiés transformés ou transformables par des séquences d'ADN clonées par la mise en oeuvre des techniques du génie génétique, ces micro-organismes modifiés étant cependant tels que la séquence d'ADN clonée se trouve en fait replacée dans un environnement génétique (intervenant par exemple au niveau de la différenciation ou de la programmation des génomes contenant une séquence d'ADN identique ou équivalente) proche des conditions naturelles qui s'avèrent en général particulièrement favorable à son expression.

Le but de la présente invention est de fournir une catégorie d'hybrides cellulaires particulièrement préférée, dérivés de cellules de singe et d'hépatocytes de singe, pour la production d'un antigène du virus de l'hépatite B, de préférence de HBs Ag ou d'un polypeptide présentant des propriétés immunogènes analogues, ces hybrides cellulaires étant d'une grande stabilité et capables de rendements de production d'antigène, notamment pour la production de vaccins, rarement sinon jamais atteints, dans les micro-organismes transformés par des techniques du génie génétique. En d'autres termes, l'invention consiste dans la sélection d'hybrides cellulaires particulièrement efficaces au sein des catégories d'hybrides cellulaires définis de façon plus générale dans la susdite demande de brevet européen.

L'hybride cellulaire selon l'invention, transformé ou transformable par une séquence d'ADN clonée contenant tout ou partie d'un gène déterminé contenu dans le génome du virus de l'hépatite B, cette séquence étant incorporée ou incorporable dans des conditions autorisant son expression dans ledit hybride cellulaire, est caractérisé en ce qu'il contient, d'une part, une partie au moins du patrimoine génétique d'une lignée d'hépatocytes de singe, de préférence de chimpanzé ou de singe vervet, naturellement favorables à l'expression de gènes naturels contenus dans le génome du virus de l'hépatite B et, d'autre part, un marqueur génétique permettant à cet hybride cellulaire de pousser dans un milieu sélectif ou contenant un principe actif normalement léthal pour des cellules de singe, mais inactivable par le polypeptide exprimé par ledit marqueur génétique. L'hybride cellulaire selon l'invention contient de préférence également une partie au moins du patrimoine génétique de cellules établies mais non tumorales ou oncogènes de singe, de préférence de cellules de la lignée VERO dont des caractéristiques particulières sont encore rappelées plus loin.

L'invention concerne également un procédé de fabrication d'un tel hybride cellulaire au sein d'un milieu de culture approprié, ce procédé étant caractérisé:

— en ce que l'on réalise une coculture, dans des conditions propres à permettre leur hybridation, d'une part, de cellules non tumorales de singe établies dans lesquelles la susdite partie de gène du génome de l'hépatite B est exprimable ou exprimée, ces cellules non tumorales étant cependant dépourvues d'une séquence génétique déterminée et, d'autre part, d'hépatocytes de singe, de préférence de chimpanzé, qui sont aptes ou ont au préalable été rendus aptes à fournir ladite séquence génétique déterminée à au moins une partie des hybrides cellulaires qui seront formés dans ledit milieu de culture, et,

— en ce que cette culture est réalisée dans un milieu qui n'autorise le développement des cellules établies de singe, que lorsque celles-ci sont complémentées par ladite séquence génétique déterminée ou appropriée apportée par les hépatocytes, et

— en ce que l'on recueille les hybrides cellulaires formés.

Tout type de cellules établies non tumorales de singe pouvant être maintenue en culture peut être utilisé pour la mise en oeuvre du procédé selon l'invention. On a de préférence recours à des lignées cellulaires hétéroploïdes Vero de primates non humains, telles que celles décrites par Petricciani, J. C., Kirschstein, R. L., Hines, J. E., Wallace, R. E. et Martin, D. P. (1973) "J. Natl. Cancer Inst.", *51*, 191—196. Ces dernières cellules se sont révélées non tumorigènes, même lorsque testées dans des singes soumis à un traitement immunosuppresseur.

Le milieu de culture, aussi bien que les cellules établies mises en oeuvre, sont choisis de façon à satisfaire aux conditions qui ont été définies plus haut. Le milieu est tel et les cellules établies sont telles que celles-ci ne peuvent pas se développer dans le milieu choisi, sauf à être complémentées par une séquence génétique apportée par les hépatocytes.

Les cellules établies sont par exemple constituées par des cellules présentant une déficience naturelle ou induites au niveau d'un de leurs gènes, cette déficience leur interdisant de se développer dans un milieu déterminé, cette déficience pouvant cependant être compensée par l'incorporation d'un gène homologue exprimable sous la forme d'une protéine homologue à celle qui aurait été spécifiée par le gène correspondant, s'il n'avait été déficient. Les cellules ayant incorporé le gène homologue sont alors à même de se développer dans le milieu considéré. C'est à cette possibilité de compensation que se réfère l'expression "complémentation" qui a été utilisée dans la définition la plus générale indiquée plus haut du procédé selon l'invention. Divers exemples de gènes ou de séquences d'ADN complémentables ont été mentionnés par exemple dans la demande de brevet France no. 81 01137 déposée le 2 mars 1981. A ce type d'ADN appartiennent notamment ceux du virus dit "Herpès simplex Virus" du type 1 (HSV1), le gène de la thymidine-kinase (TK), le gène de l'adénine phosphoribosyl-transférase (APRT) et de la dihydrofolate réductase (DHFR), etc. de l'enzyme xanthine-guanine phosphoribosyl-transférase (XGPRT), ou de l'hypoxanthine guanine phosphoribosyl transférase (HGPRT).

A chacun de ces "ADN-de complémentation" correspondent des milieux répondant aux conditions sus-indiquées. Dans le cas préféré conforme à la présente invention, dans lequel on a recours à des cellules VERO HGPRT⁻, on aura avantageusement recours au milieu connu sous le nom de "milieu HAT" (contenant de l'hypoxanthine et de l'amino-ptérine en sus de la thymidine). Ce milieu est connu pour n'autoriser l'éventuelle synthèse de thymidine phosphate que par l'intermédiaire d'une voie métabolique dite "voie de sauvetage" (salvation pathway), cette voie impliquant cependant que soit préservée l'intégrité du gène HGPRT des cellules susceptibles de s'y développer. Puisque la séquence nucléique de complémentation va leur être apportée par les hépatocytes (eux-mêmes HGPRT⁺), seuls les hybrides auxquels le gène correspondant a été transmis au cours de la fusion cellulaire peuvent se développer dans le milieu de culture choisi. Les cellules établies non engagées dans la constitution de l'hybride et non complémentées ne peuvent s'y développer, tout comme les cellules primaires elles-mêmes qui, par nature, n'ont qu'une durée de survie limitée.

Dans un mode de mise en oeuvre équivalent du procédé selon l'invention, on aura recours à l'introduction dans le milieu d'une substance susceptible d'interdire la prolifération, voire de tuer rapidement les deux types de cellules, dans la mesure où à cette substance correspond une deuxième substance, notamment une enzyme, susceptible d'inactiver la première, la seconde étant codée par une séquence d'ADN déterminée, susceptible d'être introduite préalablement dans les cellules primaires. La première substance est par exemple constituée par un antibiotique, tel que celui connu sous la désignation G418, la seconde substance étant alors constituée par un aminoglycoside 3'-phosphotransférase. On pourra encore se référer pour l'illustration de ce principe à la demande de brevet déjà mentionnée plus haut no. 81 0437. Comme dans le cas précédent, seul survivra l'hybride, qui aura "reçu en héritage" de la cellule primaire la séquence d'ADN codant pour ladite aminoglycoside 3'-phosphotransférase.

Selon une première alternative du procédé selon l'invention, soit les cellules établies, soit les hépatocytes, soit les deux à la fois, ont été transformés préalablement à la coculture susdite avec la séquence d'ADN clonée contenant un fragment du génome de l'hépatite B. Le procédé selon l'invention comprend alors une sélection, après la susdite coculture, des susdits hybrides cellulaires formés qui se révèlent aussi producteurs du peptide immunogène ou de l'antigène codé par la susdite séquence d'ADN clonée.

Dans une seconde alternative du procédé selon l'invention, ce sont les hybrides cellulaires obtenus, bien que non secréteurs, qui sont transformés par la séquence d'ADN clonée, le procédé selon l'invention comprenant alors encore la sélection, après la susdite coculture, de ceux des hybrides cellulaires formés qui se révèlent aussi être producteurs de l'antigène, notamment du type HBsAg codé par la séquence d'ADN clonée. Dans ce qui précède, l'hybride non secréteur pouvait aussi bien être constitué par l'hybride obtenu à partir d'hépatocytes et de cellules établies qui n'avaient pas elles-mêmes été transformées au préalable. Celles-ci pouvaient cependant l'avoir également été. Dans ce cas, l'hybride utilisé pour la transformation pouvait être l'un de ceux qui n'avait pas été retenu dans le cadre de la sélection des hybrides cellulaires envisagée dans la première alternative.

La séquence d'ADN clonée dérivée du génome du virus de l'hépatite B, dont l'expression est recherchée, peut être constituée par tout fragment de gène naturel ou toute séquence résultant d'une transcription réverse d'un brin d'acide nucléique monocaténaire, par exemple d'un mARN, par voie enzymatique, voire par tout ADN obtenu par synthèse chimique. Par exemple, cette séquence est constituée par un cADN codant pour un polypeptide identique à celui exprimé par le gène naturel correspondant ou un polypeptide équivalent, l'équivalence résultant notamment de l'analogie immunologique susceptible d'être reconnue entre ces deux polypeptides dans des réactions croisées correspondantes avec des anticorps préalablement formés contre le polypeptide naturel et le polypeptide équivalent. L'expression "gène", telle qu'elle est utilisée plus haut dans la définition de l'invention, doit être interprétée comme s'étendant à toute sèquence nucléotidique correspondante, telle que ci-dessus mentionnée.

Pour réaliser la transformation ou la transfection des cellules établies ou des cellules primaires, avant hybridation de celles-ci, soit encore des hybrides préalablement formés, on peut avoir recours à tout vecteur, notamment plasmide approprié. Les conditions auxquelles ce vecteur doit satisfaire sont uniquement qu'elles permetent la transformation effective et l'expression de la séquence d'ADN clonée dans les cellules utilisées. L'homme du métier sera parfaitement à même de choisir les types de plasmide à mettre en oeuvre, compte tenu de la nature des cellules à mettre en oeuvre dans l'hybridation et le cas échéant de la séquence d'ADN à cloner. Lorsque la séquence à cloner ne se trouve pas déjà placée sous le contrôle du promoteur qui permettra l'expression de cette séquence au sein de la cellule transformée, il sera avantageux de la mettre sous la contrôle direct d'un promoteur choisi parmi ceux qui permettent non seulement l'expression, mais l'excrétion du polypeptide exprimé dans le milieu de culture. Des promoteurs avantageux seront par exemple des promoteurs forts tels que ceux obtenus à partir du virus SV40 ou du virus MMTV.

Bien entendu, les vecteurs en question comprennent avantageusement tous les éléments génétiques susceptibles d'assurer la transcription et la traduction de la séquence d'ADN clonée dans une cellule de singe, par exemple les sites de polyadénylation, et de préférence aussi de la terminaison de la transcription de la séquence d'ADN concernée. Ceci étant, il n'est pas nécessaire d'insister sur la constitution des plasmides susceptibles d'être utilisés. Un grand nombre de ces plasmides ont été décrits dans la littérature technique. Ils peuvent tous être mis en oeuvre dès lors qu'ils sont reconnus capables de transformer des cellules de mammifère, plus particulièrement de cellules de la lignée VERO et d'apporter à ces dernières le gène ou la séquence d'ADN clonée susceptible d'être transcrite et exprimée dans ces cellules.

Enfin l'hybridation proprement dite des cellules primaires et établies peut être réalisée dans des conditions classiques. Elle met en oeuvre de façon en soi connue tout agent capable de fragiliser les parois cellulaires, au point de rendre la fusion possible. On peut classiquement utiliser le virus de Sendaï ou plus avantageusement encore des polyalcoylène-polyols (PEG 1000), notamment le polyéthylène-glycol, par exemple à raison de 500 mg/ml ou à raison d'une solution de 40% poids/volume de milieu de culture sans sérum du milieu de culture pendant 1 minute.

D'autres caractéristiques préférées de l'invention apparaîtront encore au cours de la description de modes de mise en oeuvre préférés du procédé selon l'invention dans l'exemple qui suit.

Stratégie suivie:
— Utilisation d'hépatocytes de singe mis en culture primaire, et fusion avec des cellules établies de singe, les cellules VERO, qui sont en outre HGPRT⁻ (pour la sélection ultérieure) et qui après intégration du gène S l'exprime à un niveau relativement faible.
— Sélection des clones hybrides, et étude de leur niveau d'expression.

Matériel et méthodes:
1) *Obtention des lignées cellulaires de singe produisant la protéine HBs, après transfection puis sélection des clones producteurs.*

Des cellules VERO HGPRT⁻ (cellules de singe dérivées de cellules VERO déficientes en hypoxanthine guanine phosphoribosyl transférase, obtenues par Lee Cl. Y. et al 1983) ont été cotransfectées (technique de Graham & Van Der Eb 1973) par de l'ADN de deux vecteurs, l'un portant la région codante pour la protéine HBs (plasmide PASV, sous type a y w), l'autre (plasmide PW) portant le gène de l'aminoglycoside-3′-phosphotransférase (APH3′). Les cellules transfectées exprimant l'enzyme APH3′ ont été sélectionnées en présence de 400 g/ml d'aminoglycoside G 418 (Colbère-Garapin et al 1980). Les clones issus de cette sélection ont alors été testés pour la production de la protéine HBs. 5 $10^5$ cellules VERO HGPRT⁻ ont été co-transfectées par 10 g d'ADN du plasmide PASV et par 2 g d'ADN du plasmide PW. Quatre jours après transfection, les cellules ont été divisées en quatre, et le milieu sélectif leur a été appliqué.

2) *Origine des ADN transfectés.*

— Le plasmide PASV est dérivé du plasmide PAC2 (Pourcel et al 1982) et porte le gène codant pour la region S du virus HBV (fragment Stu 43—BgIII 1984), l'origine de réplication du virus SV 40 ainsi que le promoteur précoce (fragment Pvu II 253—Hind III 5154). Le fragment pBR322 est délété entre 1120 et 2490 (R1 4360—Sal I 650) (Lusky M. et al 1981).

— Le plasmide PW porte le gène codant pour l'amino-glycoside-3′-phosphotranférase conférant la résistance à l'aminoglycoside G 418 aux cellules exprimant cette enzyme (Colbère-Garapin 1981).

3) *Obtention, isolement et mise en culture primaire des hépatocytes de singe.*

— Le foie d'un singe (singe vert d'Afrique) a été obtenu de l'Institut Mérieux.

— Les hépatocytes ont été isolés après perfusion du foie par la collagénase, selon la méthode de Seglen (1973), avec quelques modifications (Guguen-Guillouzo et al 1980, J. F. Houssais et N. Chenciner 1983).

— Les hépatocytes ont été mis en culture dans le milieu Ham F12, contenant Sérum Albumine, Insuline et Hydrocortisone, ainsi que 10% de Sérum de Veau foetal.

4) *Fusion des cellules VERO HGPRT⁻ (déposé le 25 Avril 1985 sous le no. I—438) (exprimant le gène S) avec les hépatocytes de singe en culture primaire.*

— La fusion a été effectuée après l'attachement des hépatocytes sur le plastique des flacons Falcon, et leur culture pendant 36 heures. 2 $10^6$ cellules des différents clones de cellules VERO HGPRT⁻ (clone 3, clone 357) ont été ajoutées aux cultures d'hépatocytes, et après leur attachement, la fusion a été induite par un court contact avec le polyéthylèneglycol, selon les modaliités décrites par R. L. Davidson et P. S. Gerald 1976); 24 heures plus tard, les culturs ont été passées dans le milieu sèlectif HAT (Littlefield 1964) afin d'éliminer les cellules parentales VERO HGPRT⁻. Les hépatocytes non fusionnés, fragilisés par le PEG ont été éliminés spontanément.

— Dans les semaines qui ont suivi, les clones hybrides viables sont isolés, remis en culture et testés quant à leur capacité de production de l'antigène HBs.

5) *Détection et dosage de la production d'antigène HBs dans le milieu de culture.*

La présence d'antigène HBs a été détectée à l'aide des tests radio-immunologiques AUS—RIA II (Abbott). La quantité d'HBs Ag excrétée dans le milieu a été déterminée par rapport à une courbe étalon établie par dilution du standard d'HBs Ag de Abbott.

Résultats:

— Deux lignées cellulaires (V3 et V357) obtenues après cotransfection des cellules VERO HGPRT⁻ ont été utilisées pour les expériences de fusion avec les hépatocytes de singe (HAS).

— Les niveaux de production de l'antigène HBS dans ces 2 lignées ont été les suivants:

— V3 à transfert 4: 36 ng/$10^6$ cellules/24 heures (c'est-à-dire 18 ng/5 $10^5$ cellules);

— V357 à transfert 7: 65 ng/$10^6$ cellules/24 heures (c'est-à-dire 32,5 ng/5 $10^5$ cellules).

Les niveaux de production de l'antigène HBs dans les clones hybrides se sont répartis de la façon suivante:

a) *Hybrides V357 × HAS*
4 clones testés:
— 190 ng/5 $10^5$ cellules/24 heures
— 169 ng/5 $10^5$ cellules/24 heures
— 95 ng/5 $10^5$ cellules/24 heures
— 70 ng/5 $10^5$ cellules/24 heures

b) *Hybrides V3 × HAS*
— 484 ng/5 $10^5$ cellules/24 heures
— 465 ng/5 $10^5$ cellules/24 heures
— 384 ng/5 $10^5$ cellules/24 heures
— 231 ng/5 $10^5$ cellules/24 heures
— 714 ng/5 $10^5$ cellules/24 heures
— 291 ng/5 $10^5$ cellules/24 heures

Au vu de ces résultats, il convient de remarquer:

— que la cellule VERO seule, après intégration du gène S, a toujours, malgré de nombreux essais dans divers laboratoires, exprimé ce gène à un niveau peu élevé,

— dans l'hybride V357 × HAS, on a obtenu un coefficient multiplicateur allant de 2 à 5,

— dans la fusion V3 × HAS, celui-ci s'est situé entre 12 et 40.

Il apparaîtra clairement à l'homme du métier que le système cellulaire hybride présente encore une grande latitude d'optimisation, par exemple en agissant au niveau du vecteur et des séquences d'ADN transfectées (promoteurs cellulaires, séquences enhancer ... — et au niveau des hybrides (sous-clonage, isolement de sous-clones hyperproducteurs ...) ou par modification de la composition du milieu de culture, par addition de molécules naturelles ou synthétiques augmentant l'expression des gènes, etc.

Dans ce qui précède, l'invention a été illustrée surtout en rapport avec la production de l'antigène HBs. Il va de soi que la technique décrite est applicable de la même façon à la production de polypeptides codés par toutes autres parties du génome du virus de l'hépatite B, par exemple à la production de l'antigène HBc. Une autre forme préférée s'adresse à des hybrides cellulaires rendus producteurs (selon les mêmes techniques que précédemment décrites) à la production de particules HBs portant la région peptidique, dite Pré S, contenant un récepteur pour la sérum albumine polymérisée.

L'hybride V3 × HAS (cellules b32c) a été déposé le 25 Avril 1985 à la Collection Nationale des Cultures de Microorganismes (CNCM) de l'Institut Pasteur de Paris, France, sous le no. I—438.

## Revendications

1. Procédé de fabrication d'un hybride cellulaire exprimant ou susceptible d'être rendu capable d'exprimer une séquence d'ADN clonée, correspondant à au moins une partie du génome du virus de l'hépatite B, caractérisé:

— en ce que l'on réalise une coculture, dans des conditions propres à permettre leur hybridation, d'une part, de cellules eucaryotes établies de singe, dans lesquelles la susdite séquence d'ADN clonée est exprimable ou exprimée et, d'autre part, d'hépatocytes de singe, de préférence de chimpanzé, cette coculture étant réalisée dans un milieu qui n'autorise pas le développement desdites cellules établies, lorsqu'elles ne sont pas complémentées par une séquence génétique appropriée apportée par les hépatocytes et

— en ce que l'on recueille les hybrides cellulaires formés.

2. Procédé selon la revendication 1, caractérisé en ce que, soit les cellules établies, soit les hépatocytes, soit les deux à la fois, avaient été transformées, préalablement à la coculture susdite, avec la séquence d'ADN clonée susdite et en ce que l'on sélectionne, après ladite coculture, ceux des hybrides cellulaires formés, qui se révèlent ainsi être producteurs du polypeptide codé par le susdit ADN cloné.

3. Procédé selon la revendication 1, caractérisé en ce que les hybrides cellulaires obtenus à l'issue de la susdite coculture sont eux-mêmes transformés avec ladite séquence d'ADN clonée et en ce que l'on sélectionne, après ladite coculture, ceux des hybrides cellulaires formés, qui se révèlent aussi être producteurs du polypeptide codé par la susdite séquence clonée.

4. Procédé selon l'une quelconque des revendications 1 à 3, caractérisé en ce que les cellules établies de singe sont des cellules de la lignée VERO.

5. Procédé selon la revendication 4, caractérisé en ce que les cellules VERO sont des cellules HGPRT⁻ et en ce que le milieu de culture est un milieu sélectif HAT.

6. Procédé selon l'une quelconque des revendications 1 à 5, caractérisé en ce que le milieu de culture contient un antibiotique, notamment du type aminoglycoside, capable de pénétrer dans des cellules eucaryotes, et en ce que les hépatocytes, ont au préalable été transformées avec une séquence d'ADN codant pour une enzyme capable d'inactiver l'antibiotique.

7. Procédé selon la revendication 6, caractérisé en ce que l'antibiotique est constitué par le G418 et en ce que la séquence d'ADN code pour une aminoglycoside 3'-phosphotransférase.

8. Procédé selon l'une quelconque des revendications 1 à 7, caractérisé en ce que la susdite séquence d'ADN clonée contient au moins une partie du gène S du virus de l'hépatite virale B.

9. Procédé selon la revendication 8, caractérisé en ce que la susdite séquence d'ADN clonée contient aussi la région pré-S du génome du virus de l'hépatite virale B.

10. Hybride cellulaire caractérisé en ce qu'il résulte de la fusion de cellules de singe établies, notamment de cellules VERO, et d'hépatocytes de singe, de préférence de chimpanzé, naturellement favorables à l'expression de gènes naturels contenus dans le génome du virus de l'hépatite B, cet hybride étant stable et capable de se multiplier, transformé ou transformable par une séquence d'ADN clonée contenant tout ou partie d'un gène déterminé contenu dans le génome du virus de l'hépatite B, cette séquence étant incorporée ou incorporable dans des conditions autorisant son expression dans ledit hybride cellulaire, et en ce qu'il contient un marqueur génétique permettant à cet hybride cellulaire de pousser dans un milieu sélectif ou contenant un principe actif normalement léthal pour des cellules de singe, mais inactivable par le polypeptide exprimé par ledit marqueur génétique.

11. Hybride cellulaire selon la revendication 10, caractérisé par le fait qu'il comporte, incorporé à son génome, une séquence d'ADN codant pour un polypeptide immunogène protecteur contre le virus de l'hépatite B.

12. Hybride cellulaire selon la revendication 11, codant pour une partie au moins du gène S et, le cas échéant, de la région pré-S du génome du virus de l'hépatite B.

## Patentansprüche

1. Verfahren zur Herstellung von Hybridzellen, die eine klonierte DNA-Sequenz exprimieren oder exprimieren können, die zumindest einem Teil des Genoms des Hepatitis-B-Virus entspricht, gekennzeichnet durch

— Cokultivierung einerseits von etablierten eukaryontischen Affenzellen, in denen die klonierte DNA-Sequenz exprimiert wird oder exprimiert werden kann, und andererseits von Hepatozyten von Affen, vorzugsweise von Schimpansen, unter Bedingungen, die ihre Hybridisierung erlauben, in einem Medium, in dem sich die etablierten Zellen nicht entwickeln können, wenn sie nicht mit einer geeigneten, von den Hepatozyten stammenden genetischen Sequenz komplementiert sind, und

— Gewinnung der gebildeten Hybridzellen.

2. Verfahren nach Anspruch 1, dadurch gekennzeichnet, daß vor der Cokultivierung entweder die etablierten Zellen oder die Hepatozyten oder beide zugleich mit der klonierten DNA-Sequenz transformiert worden sind, und nach der Cokultivierung diejenigen der gebildeten Hybridzellen selektioniert werden, die das von der klonierten DNA kodierte Polypeptid produzieren.

3. Verfahren nach Anspruch 1, dadurch gekennzeichnet, daß die nach der Cokultivierung erhaltenen Hybridzellen selbst mit der klonierten DNA-Sequenz transformiert werden und nach der Cokultivierung diejenigen der gebildeten Hybridzellen selektioniert werden, die das von der klonierten Sequenz kodierte Polypeptid produzieren.

4. Verfahren nach einem der Ansprüche 1 bis 3, dadurch gekennzeichnet, daß die etablierten Affenzellen Zellen der Linie VERO sind.

5. Verfahren nach Anspruch 4, dadurch gekennzeichnet, daß die VERO-Zellen HGPRT$^-$Zellen sind, und das Kulturmedium ein selektives HAT-Medium ist.

6. Verfahren nach einem der Ansprüche 1 bis 5, dadurch gekennzeichnet, daß das Kulturmedium ein Antibiotikum, insbesondere ein Aminoglycosid, das in die eukaryotischen Zellen eindringen kann, enthält und daß die Hepatozyten vorher mit einer DNA-Sequenz, die für ein Enzym kodiert, das das Antibiotikum inaktivieren kann, transformiert worden sind.

7. Verfahren nach Anspruch 6, dadurch gekennzeichnet, daß das Antibiotikum G418 ist und die DNA-Sequenz für eine Aminoglycosid-3'-Phosphotransferase kodiert.

8. Verfahren nach einem der Ansprüche 1 bis 7, dadurch gekennzeichnet, daß die klonierte DNA-Sequenz zumindest einen Teil des S-Gens des Virus der viralen Hepatitis B enthält.

9. Verfahren nach Anspruch 8, dadurch gekennzeichnet, daß die klonierte DNA-Sequenz auch den Prä-S-bereich des Genoms des Virus der viralen Hepatitis B enthält.

10. Hybridzelle, dadurch gekennzeichnet, daß sie das Ergebnis einer Fusion von etablierten Affenzellen, insbesondere VERO-Zellen, und Hepatozyten von Affen, vorzugsweise von Schimpansen, ist, die natürlicherweise für eine Expression natürlicher, im Genom des Hepatitis-B-Virus enthaltener Gene günstig sind, wobei diese Hybridzelle stabil und reproduktionsfähig ist, mit einer klonierten DNA-Sequenz, die das Ganze oder Teile eines bestimmten, im Genom des Hepatitis-B-Virus enthaltenen Gens enthält, transformiert wurde oder transformiert werden kann, wobei diese Sequenz unter Bedingungen, die ihre Expression in der Hybridzelle erlauben, eingebracht wird oder eingebracht werden kann, und ein Markergen enthält, das das Wachsen der Hybridzelle in einem selektiven Medium oder in einem Medium ermöglicht, das einen normalerweise für Affenzellen tödlichen Wirkstoff enthält, der aber durch das vom Markergen exprimierte Polypeptid inaktiviert werden kann.

11. Hybridzelle nach Anspruch 10, dadurch gekennzeichnet, daß sie in ihrem Genom eine DNA-Sequenz enthält, die für ein immunogenes Polypeptid, das Schutz gegenüber dem Hepatitis-B-Virus gewährt, kodiert.

12. Hybridzelle nach Anspruch 11, die zumindest einen Teil des S-Gens und gegebenenfalls des Prä-S-Bereichs des Genoms des Hepatitis-B-Virus kodiert.

## Claims

1. A process for producing a cellular hybrid expressing or susceptible of being rendered capable of expressing a sequence of cloned DNA, corresponding to at leat part of the genome of the hepatitis B virus, characterized:
   — in that a co-culture is carried out, under conditions appropriate to allow their hybridization, on the one hand, of established eukaryotic cells of monkey, in which the aforesaid cloned DNA sequence is expressible or expressed and, on the other hand, monkey hepatocytes, preferably from the chimpanzee, this co-culture being carried out in a medium which does not permit the development of the said established cells when they are not complemented by an appropriate genetic sequence, and
   — in that the hybrid cells formed are collected.

2. The process according to claim 1, characterized in that the established cells, or the hepatocytes, or both at once, have been transformed, prior to the aforesaid co-culture, by the aforesaid cloned DNA sequence, and characterized in that, after the said co-culture, those of the said cellular hybrids formed, which thus proved to be producers of the polypeptide coded by the aforesaid cloned DNA, are selected.

3. The process according to claim 1, characterized in that the cellular hybrids obtained as a result of the aforesaid co-culture are themselves transformed with the said cloned DNA sequence and characterized in that after the said co-culture, those of the said cellular hybrids formed, which also prove to be producers of the polypeptide coded by the aforesaid cloned DNA, are selected.

4. The process according to any one of the claims 1 to 3, characterized in that the established monkey cells are cells of the VERO line.

5. The process according to claim 4, characterized in that the VERO cells are HGPRT$^-$ cells and whereby the culture medium is a selective HAT medium.

6. The process according to any one of the claims 1 to 5, characterized in that the culture medium contains an antibiotic, notably of the aminoglycoside type, capable of penetrating into eukaryotic cells, and whereby the hepatocytes have first been transformed with a DNA sequence coding for an enzyme capable of inactivating the antibiotic.

7. The process according to claim 6, characterized in that the antibiotic is constituted of G418 and characterized in that the DNA sequence codes for an aminoglycoside 3'-phosphotransferase.

8. The process according to any one of the claims 1 to 7, characterized in that the aforesaid cloned DNA sequence contains at least a part of the S gene of the virus of viral hepatitis B.

9. The process according to claim 8, characterized in that the aforesaid cloned DNA sequence also contains the pre-S region of the genome of the virus of viral hepatitis B.

10. A cellular hybrid, characterized in that it results from the fusion of established cells of monkey, notably VERO cells, with monkey hepatocytes, preferably from the chimpanzee, naturally favorable to the expression of natural genes contained in the genome of the hepatitis B virus, this hybrid being stable and capable of multiplying, transformed or transformable by a cloned DNA sequence containing all or part of a given gene contained in the genome of the hepatitis B virus, this sequence being incorporated or capable of being incorporated under conditions permitting its expression in the said cellular hybrid, and characterized in that it contains a genetic marker permitting this cellular hybrid to grow in a selective medium or one containing an active principle normally lethal to the monkey cells, but inactivatible by the polypeptide expressed by the said genetic marker.

11. The cellular hybrid according to claim 10, characterized in that it contains, incorporated into its genome, a DNA sequence coding for an immunogenic polypeptide protective against the virus of hepatitis B.

12. The cellular hybrid according to claim 11, coding for at least a part of the S gene and, as the case may be, of the pre-S region of the genome of the hepatitis B virus.